# EUROPÄISCHE PATENTANMELDUNG

(11) **EP 1 997 458 A1**
(43) Veröffentlichungstag der Anmeldung: **03.12.2008**
(21) Anmeldenummer: 08008887.5
(22) Anmeldetag: 14.05.2008
(51) Int. Cl.: A61F 2/88, A61F 2/86, A61F 2/90

(54) **Medizinisches Implantat, insbesondere Stent**

(30) Priorität: 02.06.2007 DE 102007025921
(71) Anmelder: BIOTRONIK VI Patent AG, 6341 Baar (CH)
(72) Erfinder: Lootz, Daniel, 18119 Rostock (DE); Winter, Alexander, 18057 Rostock (DE)
(74) Vertreter: Lindner-Vogt, Karin L.

(57) **Zusammenfassung**

Ein medizinisches Implantat, insbesondere Stent, ist mit einer aus verformbaren Streben (2) zusammengesetzten Grundstruktur (1) versehen, die vorzugsweise aus einem röhrenförmigen Rohling (3) durch Laserschneiden herausgearbeitet ist. Es ist eine an die Streben (2) der Grundstruktur (1) angebundene Feinstruktur (4) aus einem draht- oder fadenartigen Material vorgesehen.

## Beschreibung

Die Erfindung betrifft ein medizinisches Implantat und insbesondere einen Stent mit einer aus verformbaren Streben zusammengesetzten Grundstruktur, die vorzugsweise aus einem röhrenförmigen Rohling durch Laserschneiden herausgearbeitet ist. Alternative Herstellungsmethoden, wie beispielsweise durch Lasersinter Rapid Prototyping, sind ebenfalls denkbar.

Derartige medizinische Implantate sind in unterschiedlichen Ausführungsformen bekannt, wobei die herkömmlicherweise lasergeschnittenen Streben in ihrer Grundstruktur auf Formelemente, wie Ringsegmente, Spiralen, Axialverbinder usw., zurückgreifen. Die Verbindung zwischen diesen verschiedenen Strebenstrukturen ist aufgrund des Herstellungsverfahrens prinzipbedingt stoffschlüssig. Dies bedeutet, dass die für den medizinischen Einsatz notwendigen Formänderungen des Stents beispielsweise beim Applizieren aus einem Katheter heraus durch eine Verformung des Stentwerkstoffs selbst zu realisieren sind. Derartige Verformungen können elastisch oder auch plastisch vonstatten gehen, wobei die Grenzen für eine solche Verformung werkstoffabhängig und damit ggf. sehr stark eingeschränkt sind. Des Weiteren besitzen stoffschlüssige Verbindungen die Eigenschaft, Kräfte sowohl in Druck- als auch in Zugrichtung zu übertragen. Bei relativ geringen Verformungen im elastischen Bereich sind hierbei die Übertragungseigenschaften richtungsunabhängig. Eine beispielsweise in Druckrichtung sehr flexible Verbindung besitzt bei geringen Verformungen zwangsläufig auch in Zugrichtung eine hohe Flexibilität. Dasselbe gilt für steife Verbindungen. Beispielhaft kann in diesem Zusammenhang die US 5,102,417 A genannt werden. Die mechanischen Eigenschaften derartig aufgebauter Stents können durch die stoffschlüssige Verbindung der Strebenstrukturen also nur in begrenztem Umfang in Abhängigkeit der Belastungsrichtung gestaltet werden. Nachteilig kann sich dies beispielsweise auf die Eigenschaften eines Stents unter Biegebelastung auswirken. Hier kann druckseitig ein sehr weiches Verformungsverhalten, zugseitig jedoch eine geringe Verformung erwünscht sein, um bei insgesamt niedriger Biegesteifigkeit gleichzeitig eine dem Blutgefäß möglichst gut angepasste, röhrenförmige Außenkontur beizubehalten.

Eine weitere Bauform medizinischer Implantate, insbesondere von Stents, stellen geflochtene Stents dar. Sie sind beispielsweise aus US 7,001,425 B2 oder US 6,342,068 B1 bekannt. Die geflochtene Struktur ohne stoffschlüssige Verbindungen ermöglicht bei diesen Implantaten große Relativbewegungen zwischen sich kreuzenden Drähten, was den Stents eine vergleichsweise hohe Flexibilität verleiht. Die aufnehmbaren Radialkräfte sind bei diesem Stenttyp vergleichsweise gering. Es tritt prinzipbedingt meist eine ausgeprägte Längenänderung bei Stentaufweitung bzw. Kompression auf, was im Allgemeinen unerwünscht ist. Des Weiteren ist eine Biegung der Stents mit einer relativ starken Reduktion der durchströmbaren Querschnittsfläche verbunden.

Eine Besonderheit stellen rückziehbare Stents dar. Sie zeichnen sich dadurch aus, dass sie nach einer teilweisen Freisetzung aus dem Einführsystem wieder in selbiges zurückgezogen werden können, um beispielsweise eine neue Positionierung im Gefäß vorzunehmen. Ein Stent dieses Typs ist beispielsweise aus US 7,037,331 B2 bekannt. Um die Rückführbarkeit zu gewährleisten, muss die Struktur eines solchen Stents vollvernetzt sein, so dass keine freien Enden innerhalb der Struktur existieren, die sich beim Zurückführen in das Freisetzsystem verhaken können. Dieser Umstand macht derartig aufgebaute Stents vergleichsweise biegesteif, was eine gute Anpassung an stark gewundene Gefäße erschwert.

Ein offenzelliger Stent, wie er in der US 7,037,330 B1 beschrieben ist, besitzt zwar eine niedrigere Biegesteifigkeit, kann aber aufgrund der freien Enden innerhalb der Struktur nicht zurückgezogen werden und neigt unter Biegebeanspruchung dazu, stark von der erwünschten röhrenförmigen Außenkontur abzuweichen.

Ausgehend von den geschilderten Problemen des Standes der Technik liegt der Erfindung die Aufgabe zugrunde, ein medizinisches Implantat und insbesondere einen Stent mit einer aus verformbaren Streben zusammengesetzten Grundstruktur so zu verbessern, dass unter Aufrechterhaltung vernünftiger aufnehmbarer Radialkräfte eine hohe Flexibilität in Biegerichtung bei optimaler Flächenabdeckung und Vernetzungsdichte gewährleistet wird, ohne dabei die röhrenförmige Außenkontur zu verlieren. Des Weiteren ermöglicht die Erfindung rückführbare Stents mit stark verbesserter Biegeflexibilität.

Diese Aufgabe wird durch die im Kennzeichnungsteil des Anspruches 1 angegebenen Merkmale gelöst, wonach die aus verformbaren Streben stoffschlüssig zusammengesetzte Grundstruktur ergänzt wird durch eine an diese Streben angebundene Feinstruktur aus einem draht- oder fadenartigen Material.

Durch diese Kombination lassen sich die Vorteile von lasergeschnittenen Implantaten, die Verformungen nur durch vergleichsweise hohe elastische oder plastische Beanspruchung des Werkstoffs ermöglichen, mit den Vorteilen geflochtener bzw. gewobener Implantate kombinieren. Damit wird auch die Voraussetzung für einen rückführbaren Stent mit gleichzeitig geringer Biegesteifigkeit geschaffen.

Gemäß bevorzugten Ausführungsformen der Erfindung weist die Feinstruktur mindestens einen Draht oder Faden auf, der an mindestens einer Anbindungsstelle lose, formschlüssig, stoffschlüssig, durch Verklebung oder durch einen Fixierfaden mit der Grundstruktur verbunden ist. Aufgrund der Auswahlmöglichkeit unter verschiedenen Anbindungen zwischen Feinstruktur und Grundstruktur kann der erfindungsgemäße Stent besonders gut an den gewünschten Einsatzzweck angepasst werden. Weitere bevorzugte Ausführungsformen betreffen konstruktive Details bezüglich der Verbindung von Draht oder Faden mit der Grundstruktur, in deren Zusammenhang auf die Beschreibung der Ausführungsbeispiele zur Vermeidung unnötiger Wiederholungen verwiesen wird.

Weitere bevorzugte Ausführungsformen betreffen die Zuordnung der Feinstruktur zu einer Grundstruktur aus helikal umlaufenden, mäanderförmigen Streben. Auch hier wird zur Vermeidung unnötiger Wiederholungen auf die entsprechenden Passagen der Beschreibung der Ausführungsbeispiele verwiesen.

Gemäß einer weiteren bevorzugten Ausführungsform weist der die Feinstruktur des Implantates bildende Draht oder Faden selbst eine Mikrostruktur zur Erweiterung seiner Funktionalität auf. So können Draht oder Faden aus zwei oder mehr Materialien aufgebaut sein, so dass beispielsweise mehrkomponentige Schicht- oder Verbundsysteme relativ einfach hergestellt werden können. Draht- oder fadenförmige Strukturen haben nämlich den Vorteil, dass sie in einer Endlos-Fertigung als Halbzeuge hergestellt und anschließend in der entsprechenden Konfiguration in das Implantat eingebaut werden können. So sind beispielsweise zu nennen eine Kombination aus einem stabilen bio-kompatiblen Metall mit einem abbaubaren Polymer, das als wirkstoffbeladene Beschichtung als Medikamentendepot wirkt. Sofern es sich um einen Faden handelt, kann dieser wiederum polymergetränkt sein, was gegenüber dem Aufbringen einer Wirkstoffkombination auf der Oberfläche einer metallischen Struktur den Vorteil einer besseren Inkorporierbarkeit mit sich bringt.

Weitere Materialkombinationen können beispielsweise die Beschichtung der Draht- oder Fadenoberfläche mit Siliziumkarbid zur Verbesserung der Bio-Kompatibilität oder den Einbau eines röntgendichten Werkstoffes in den Draht oder Faden betreffen. So kann beispielsweise ein Draht mit einer Beschichtung oder einem Kern aus röntgendichtem Werkstoff vorgesehen werden.

Gemäß einer weiteren Ausführungsform der Erfindung kann der Draht oder Faden als Zugelement zur Rückführung des Implantats in einen kontrahierten Zustand ausgebildet sein. In diesem Zusammenhang ist von Vorteil, dass für die Rückführung des Implantats kein gesondertes Konstruktionselement notwendig ist, da die Feinstruktur in Form des Drahtes oder Fadens, der Teil des Implantats ist, lediglich vom Implantat aus fortgeführt werden muss und als Angriffselement zur Rückführung des Implantats herangezogen werden kann.

Weitere Merkmale, Einzelheiten und Vorteile der Erfindung ergeben sich aus der nachfolgenden Beschreibung, in der Ausführungsbeispiele anhand der beigefügten Zeichnungen näher erläutert werden. Es zeigen:
- Fig. 1 bis 5: schematische Ansichten von Stents mit Feinstrukturen in unterschiedlichen Ausführungsformen,
- Fig. 6 bis 14: Ansichten von Anbindungspunkten zwischen der Fein- und Grobstruktur eines Stents in unterschiedlichen Ausführungsformen, und
- Fig. 15 bis 23: geschnittene Perspektivdarstellungen von Fäden bzw. Drähten der Feinstruktur des Stents in unterschiedlichen Ausführungsformen.

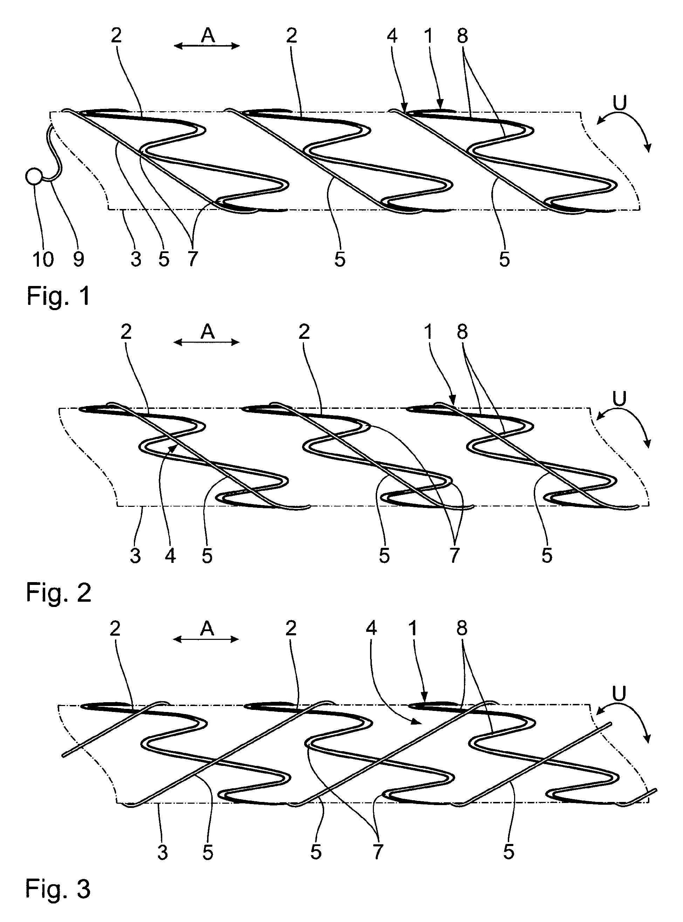

In den Fig. 1 bis 5 ist ein Stent gezeigt, der in seiner Grundstruktur 1 aus mäanderförmigen Streben 2 aufgebaut ist, die aus einem in den Fig. 1 bis 5 angedeuteten Rohling 3 durch Laserschneiden herausgearbeitet wurden. Die Grundstruktur 1 umschreibt dabei einen zylindrischen Raum, wobei die Streben 2 helikal in Umfangsrichtung U umlaufen. Die Streben 2 sind aufgrund ihres mäanderförmigen Verlaufs in bekannter Weise beispielsweise durch einen Katheterballon zur Applikation des Stents etwa in einem Koronargefäß des Herzens radial aufweitbar.

Der Grundstruktur 1 ist eine als Ganzes mit 4 bezeichnete Feinstruktur überlagert, die in den Fig. 1 bis 5 in verschiedenen Konfigurationen angelegt ist. Grundsätzlich besteht sie aus einem Draht/Faden 5, der an die Grundstruktur 1 in noch näher zu erläuternder Weise angebunden ist.

Bei der Ausführungsform gemäß Fig. 1 verläuft der Draht/Faden 5 jeweils helikal um den Stent herum und zwar entlang jeweils der Zenite 7 der Mäanderbögen 8. Der Draht/Faden 5 ist dabei am einen Ende des Stents über die Streben 2 hinaus verlängert, an seinem überstehenden Ende 9 ist ein Koppelelement 10 angebracht, an dem ein Rückzugsmechanismus für ein radiales Kontrahieren des Stents angreifen kann.

Bei der Ausführungsform gemäß Fig. 2 verläuft der Draht/Faden 5 mittig zwischen den Zeniten 7 ebenfalls helikal um den Stent herum. Bei den Ausführungsformen gemäß Fig. 1 und 2 verläuft der Draht/Faden 5 also parallel zu den Helixwindungen der Streben 2.

In Fig. 3 ist eine Ausführungsform gezeigt, bei der die Windungen der Streben 2 und der ebenfalls helixförmig umlaufende Draht/Faden 5 in gegenläufiger Richtung verlaufen, so dass sich Streben 2 und Draht/Faden 5 wabenartig kreuzen.

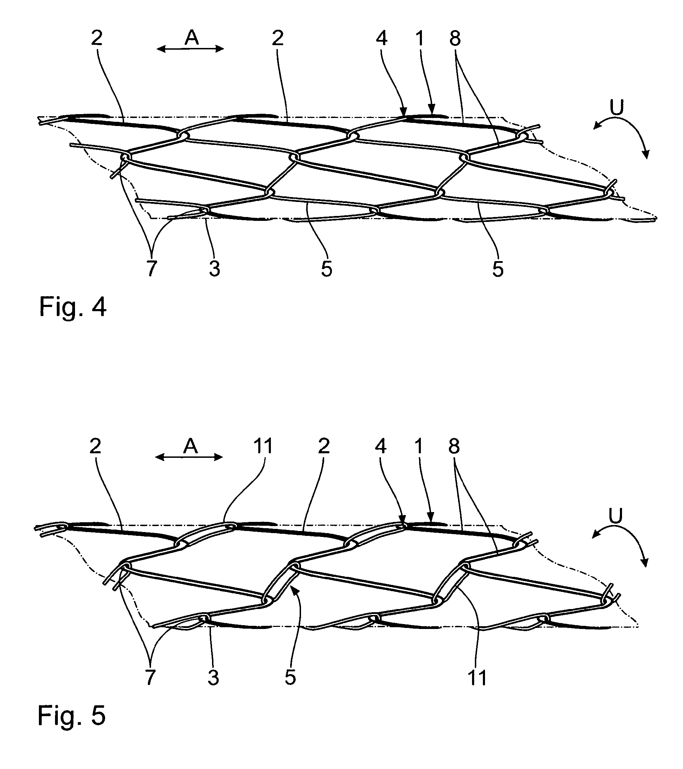

In einer weiteren Alternative, wie sie Fig. 4 zu entnehmen ist, wird ein Faden / Draht 5 zwischen den Zeniten 7 zweier benachbarter Windungen der Streben 2 zick-zack-förmig hin und her geführt, so dass die benachbarten Windungen miteinander verwoben werden.

In Fig. 5 schließlich ist eine Ausführungsform gezeigt, in der zwischen den Zeniten 7 zweier benachbarter Windungen der Streben 2 jeweils ein Faden / Draht 5 als Fixierring 11 herumgeschlungen ist.

Es ist zu ergänzen, dass bei allen Ausführungsformen gemäß den Fig. 1 bis 5 nicht näher dargestellte Axialverbinder in Axialrichtung A zwischen zwei nebeneinander in Umfangsrichtung verlaufenden Streben 2 vorgesehen sein können, falls dies aus Gründen einer besonderen Strukturstabilität des Stents notwendig ist.

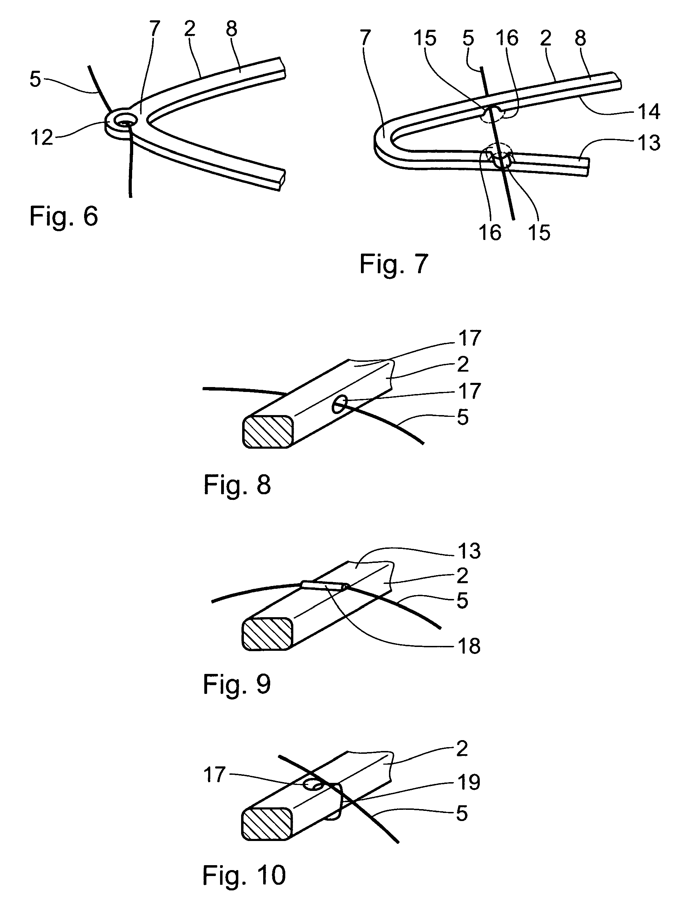

Anhand der Fig. 6 bis 14 werden nun die verschiedenen Möglichkeiten erörtert, wie ein Draht/Faden 5 an eine Strebe 2 der Grundstruktur 1 angebunden werden kann. So zeigt Fig. 6 eine Öse 12 am Zenit 7 eines Mäanderbogens 8 der Strebe 2, durch die der Draht/Faden 5 lose hindurch läuft.

In Fig. 7 läuft der Draht/Faden 5 etwa mittig über zwei benachbarte Abschnitte eines Mäanderbogens 8 in einer in entgegengesetzte Richtungen auf der Ober- bzw. Unterseite 13, 14 eingeformten Vertiefung 15. Diese ist als im Querschnitt halbzylindrische, dreieckige oder viereckige Kerbe ausführbar. Wie in Fig. 7 gestrichelt angedeutet ist, kann der Draht/Faden 5 auf der Strebe 2 auch beispielsweise durch eine Verklebung 16 so fixiert werden, dass eine Relativbewegung zwischen Strebe 2 und Draht/Faden 5 nicht möglich ist.

Fig. 8 zeigt eine Durchführung eines Drahtes / Fadens 5 durch eine Bohrung 17 in der Strebe 2.

Für eine stoffschlüssige Anbindung von Draht/Faden 5 an Strebe 2 sorgt die in Fig. 9 erkennbare Verschweißung / Verlötung / Verklebung oder anders geartete Fügetechnik 18 auf der Oberseite 13 der Strebe 2.

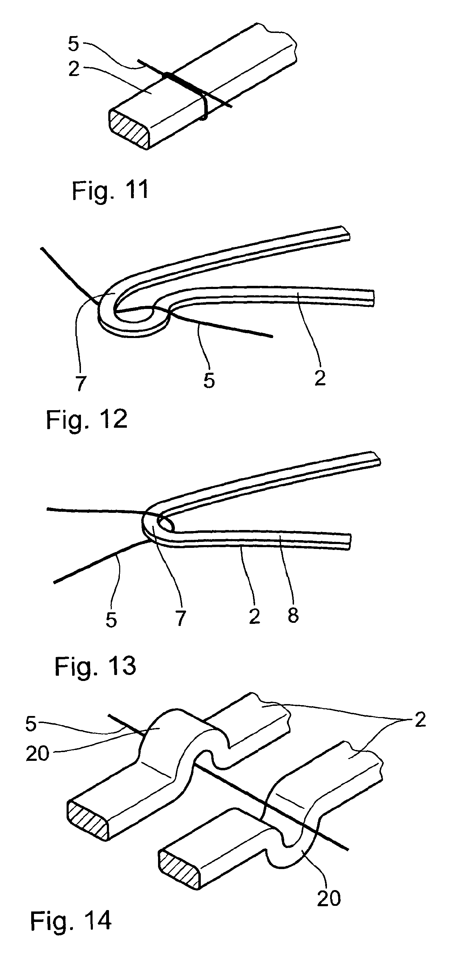

In Fig. 10 ist der Draht/Faden 5 auf die Strebe 2 im Bereich einer Bohrung 17 gelegt, durch die dann ein Fixierfaden / Fixierdraht 19 gezogen und damit der Draht/Faden 5 auf der Strebe 2 geführt wird. In Fig. 11 ist der Draht/Faden 5 um die Strebe 2 herum geschlungen.

Bei der Variante gemäß Fig. 12 sorgt eine spezielle ösenartige Ausformung der Strebe 2 im Bereich ihres Zenits 7 für eine gute Führung des Drahtes / Fadens 5.

Fig. 13 zeigt den Verlauf des in Fig. 4 dargestellten, zick-zack-förmig angelegten Drahtes /Fadens 5 im Bereich des Zenits 7 eines Mäanderbogens im Detail.

Bei der Ausführungsform gemäß Fig. 14 weisen die Streben 2 jeweils Ausformungen 20 auf, in die der Faden 6 eingelegt ist.

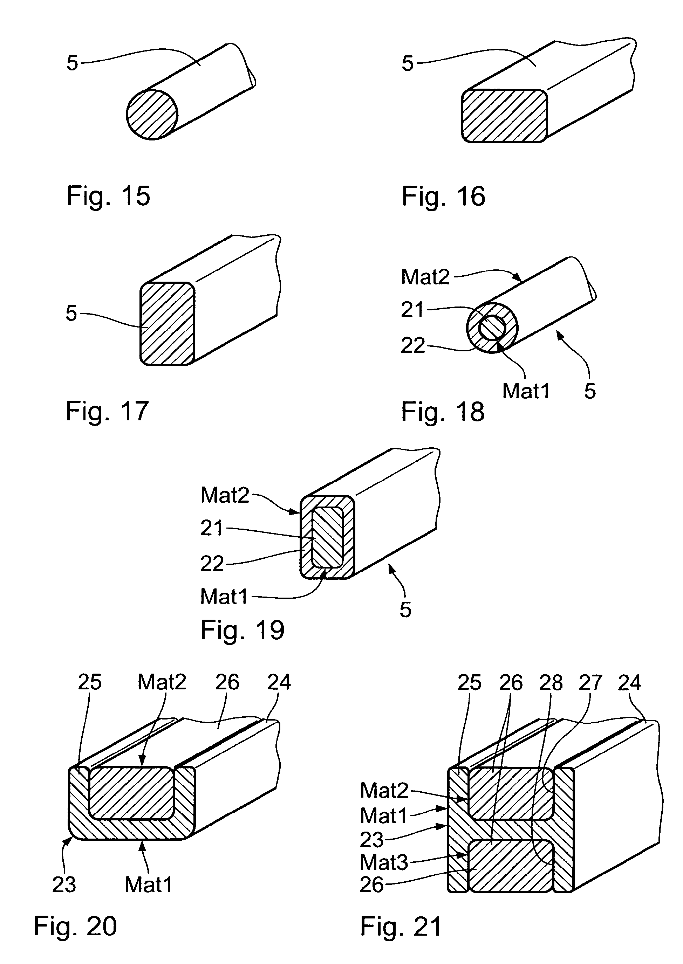

Aus den Fig. 15 bis 23 werden verschiedene Ausführungsformen des Drahtes / Fadens 5 bezüglich einer darin integrierten Mikrostruktur deutlich.

Fig. 15 zeigt die einfachste Ausführungsform, bei der ein Faden 5 beispielsweise aus einem biologisch abbaubaren Polymermaterial besteht. Entsprechend kann auch ein beständiger oder abbaubarer Draht eingesetzt werden.

Die Fig. 16 und 17 zeigen Drähte 5, die einen flach-rechteckigen Querschnitt aufweisen. Derartige Drähte 5 sind aufgrund der ebenen Oberfläche wirkungsvoll mit einem glatten Oberflächenfinish, beispielsweise durch Beizen oder Elektropolieren, zu versehen. Aufgrund ihrer richtungsabhängigen Biegesteifigkeit können die Drähte 5 gemäß Fig. 16 und 17 zur Beeinflussung des Biegeverhaltens des gesamten Stents mit bestimmter Einbaurichtung an die Grundstruktur 1 angebunden werden.

Die Fig. 18 bis 21 sowie 23 zeigen Varianten, bei denen zwei unterschiedliche Materialien miteinander kombiniert sein können. So ist bei der Ausführungsform gemäß Fig. 18 ein runder Draht 5 mit einem Kern 21 aus medizinischem Stahl (oder Nitinol) versehen, der mit einer Hülle 22 aus einem bio-kompatiblen oder biologisch abbaubaren Polymer versehen ist. Fig. 19 zeigt einen entsprechenden Aufbau bei einem Draht 5 mit flachrechteckigem Querschnitt.

Bei der Ausführungsform gemäß Fig. 20 ist ein tragendes Profil 23 in U-Form vorgesehen, zwischen dessen Schenkel 24, 25 ein zweites Material, beispielsweise ein Medikamentendepot 26 in Form von in ein abbaubares Polymer eingebetteten Wirkstoffen integriert sein.

Bei der in Fig. 21 gezeigten Ausführungsform ist das tragende Profil 23 H-förmig, so dass zwei Aufnahmenuten 27, 28 gebildet werden, die wiederum mit entsprechenden weiteren Materialien gefüllt sein können.

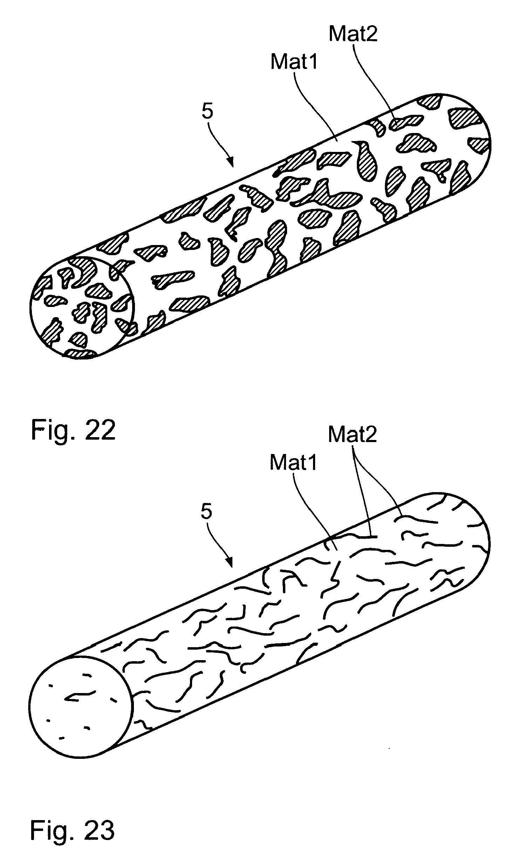

Fig. 22 zeigt einen porösen Draht/Faden 5, der offen- oder geschlossenzellige Hohlräume aufweist, die unbefüllt sein können oder einen weiteren Werkstoff (Mat2) zur Funktionserweiterung enthalten können. Fig. 23 zeigt eine faser- oder partikelverstärkte faden- oder drahtförmige Struktur 5.

Aus der nachfolgenden Tabelle sind im Übrigen Materialkombinationen dreier Materialien Mat1, Mat2, Mat3 entnehmbar, wie sie bei den Ausführungsformen gemäß Fig. 18 bis 23 realisiert werden können.

| | **Mat 1** | **Mat 2** | **Mat 3** |
|---|---|---|---|
| **Fig. 18,19****:** | biokompatibles Metall, z.B. Nitinol | Platin, Tantal, ... | |
| | | - röntgendichter Werkstoff | |
| | biokompatibles Metall | Polymer | |
| | | | |
| **Fig. 20****:** | biokompatibles Metall | Polymer | |
| | biokompatibles Metall | Platin, Tantal, ... | |
| | | - röntgendichter Werkstoff | |
| | | | |
| **Fig. 21****:** | biokompatibles Metall | Polymer - Wirkstoff / Wirkstoffkombination 1 | Polymer - Wirkstoff / Wirkstoffkombination 1 |
| | biokompatibles Metall | Polymer - Wirkstoff / Wirkstoffkombination 1 | Polymer - Wirkstoff / Wirkstoffkombination 2 |
| | biokompatibles Metall | Polymer - Wirkstoff / Wirkstoffkombination 1 | Platin |
| | biokompatibles Metall | Platin, Tantal,... Polymer | Polymer |
| | | -röntgendichter Werkstoff | |
| **Fig. 22** | biokompatibles Metall | Polymer-Wirkstoff / Wirkstoffkombination | |
| | biokompatibles Polymer | Polymer - Wirkstoff / Wirkstoffkombination | |
| | biokompatibles Metall | | |
| | biokompatibles Polymer | | |
| | biokompatibles Polymer | Wirkstoff / Wirkstoffkombination | |
| **Fig. 23** | biokompatibles Metall 1 | biokompatibles Metall 2 | |
| | biokompatibles Polymer 1 | biokompatibles Polymer 2 | |
| | biokompatibles Metall | verstärkende Faser | |
| | biokompatibles Polymer | verstärkende Faser | |

| | | | |
|---|---|---|---|
| ("Metall" in der vorstehenden Tabelle umfasst Metalle und Metalllegierungen.) | | | |

Die Legierung "Nitinol" ist dabei ein superelastischer Strukturwerkstoff. Platin dient zur Verbesserung der Röntgensichtbarkeit des Stents. Die erwähnten Polymere können sowohl abbaubar, als auch langzeitstabil ausgeführt sein. Sie können - wie oben anhand der Zeichnungen schon erläutert - als Wirkstoffträger für sogenannte "Drug-eluting-Anwendungen" dienen.

Für den Strukturwerkstoff (Mat1) kann auch ein nicht-superelastisches Material zum Einsatz kommen, wie beispielsweise Chrom-Nickel- und Kobalt-Chrom-Legierungen, Tantal, Titan, Niob und deren Legierungen.

## Patentansprüche

1. Medizinisches Implantat, insbesondere Stent, mit einer aus verformbaren Streben (2) stoffschlüssig zusammengesetzten Grundstruktur (1), die vorzugsweise aus einem röhrenförmigen Rohling (3) durch Laserschneiden herausgearbeitet ist, **gekennzeichnet durch** eine an die Streben (2) der Grundstruktur (1) angebundene Feinstruktur (4) aus einem draht- oder fadenartigen Material.

2. Implantat nach Anspruch 1, **dadurch gekennzeichnet, dass** die Feinstruktur (4) mindestens einen Draht oder Faden (5) aufweist, der an mindestens einer Anbindungsstelle lose, formschlüssig, stoffschlüssig, durch Verklebung (16) oder durch einen Fixierfaden (19) mit der Grundstruktur (1) verbunden ist.

3. Implantat nach Anspruch 2, **dadurch gekennzeichnet, dass** der Draht oder Faden (5) verschiebbar in mindestens einer Aufnahme (12, 15, 17) an der Grundstruktur (1) geführt ist.

4. Implantat nach Anspruch 3, **dadurch gekennzeichnet, dass** die Aufnahme eine kerbenförmige Vertiefung (15), Bohrung (17) oder Öse (12) an einer jeweiligen Strebe (2) der Grundstruktur (1) ist.

5. Implantat nach einem der vorgenannten Ansprüche, **dadurch gekennzeichnet, dass** die Grundstruktur (1) aus helikal umlaufenden, mäanderförmigen Streben (2) gebildet ist, wobei mindestens ein Draht oder Faden (5) der Feinstruktur (4) im Bereich der Zenite (7) der Mäanderbögen (8) oder mittig daran angebunden sind.

6. Implantat nach einem der vorgenannten Ansprüche, **dadurch gekennzeichnet, dass** die Grundstruktur (1) aus helikal umlaufenden, mäanderförmigen Streben (2) gebildet ist, wobei der mindestens eine Draht oder Faden (5) der Feinstruktur (4) an die Grundstruktur (1) parallel zu den Windungen der Streben (2), in einer gegenläufigen Helixanordnung, zwischen zwei benachbarten Windungen der Streben (2) zick-zack-förmig dazwischen verlaufend oder als zwei Windungen von Streben (2) an benachbarten Zeniten (7) miteinander verbindende Fixierringe (11) angebunden ist.

7. Implantat nach einem der vorgenannten Ansprüche, **dadurch gekennzeichnet, dass** der Draht oder Faden (5) eine Mikrostruktur zur Erweiterung seiner Funktionalität aufweist.

8. Implantat nach Anspruch 7, **dadurch gekennzeichnet, dass** der Draht oder Faden (5) aus zwei oder mehr Materialien (Mat1, Mat2, Mat3) aufgebaut ist, die ausgewählt sind aus der Gruppe stabiles oder abbaubares, biokompatibles Metall, stabiles oder abbaubares Polymer mit oder ohne Medikamentendepot, Silizium als Beschichtungsmaterial, röntgendichtes Material als Beschichtungs- oder Kernmaterial.

9. Implantat nach einem der vorgenannten Ansprüche, **dadurch gekennzeichnet, dass** der Draht oder Faden (5) als Zugelement (9, 10) zur Rückführung des Implantats in einen kontrahierten Zustand ausgebildet ist.
